# EUROPÄISCHE PATENTSCHRIFT

(11) **EP 1 537 828 B1**
(45) Veröffentlichungstag und Bekanntmachung des Hinweises auf die Patenterteilung: **08.07.2009**
(21) Anmeldenummer: 04025437.7
(22) Anmeldetag: 26.10.2004
(51) Int. Cl.: A61B 17/28

(54) **Medizinisches Instrument mit Kupplung für Instrumenteneinsatz**
Medical instrument comprising coupling for instrument insert
Dispositif medical comprenant accouplement pour un instrument emmanchable

(30) Priorität: 06.12.2003 DE 10357103
(43) Veröffentlichungstag der Anmeldung: 08.06.2005
(73) Patentinhaber: Richard Wolf GmbH, 75438 Knittlingen (DE)
(72) Erfinder: Prestel, Stephan, 76287 Rheinstetten-Mörsch (DE); Knodel, Frank, Dipl.-Ing., 75438 Knittlingen (DE); Wagner, Carl-Sebastian, 75015 Bretten (DE); Bartolic, Josef, 76229 Karlsruhe-Grötzingen (DE)
(74) Vertreter: Hemmer, Arnd

(56) Entgegenhaltungen:
- WO-A-01/01870
- DE-A1- 3 934 610
- DE-A1- 19 514 098
- DE-A1- 19 918 638
- US-A- 4 577 875
- US-A- 5 505 737

## Beschreibung

Die Erfindung betrifft ein medizinisches Instrument mit einem Instrumenteneinsatz und einer Instrumentenhandhabe, beispielsweise eine medizinische Zange.

Aus dem Stand der Technik, beispielsweise DE 198 09 120 C1, sind medizinische Instrumente bekannt, bei welchen ein Instrumenteneinsatz lösbar mit einer Instrumentenhandhabe verbindbar ist. Bei dem aus DE 198 09 120 C1 offenbarten Instrument wird der Instrumenteneinsatz mit der Instrumentenhandhabe über eine Kugelrastverbindung verbunden. Zum Verriegeln der Kugelrastverbindung ist ein mit einem Griff verbundener Einsatz vorgesehen, welcher durch eine Druckfeder in einer gesicherten Position gehalten wird, in welcher der Einsatz die Rastkugel blockiert. Zum Lösen der Verbindung wird der an der Instrumentenhandhabe vorgesehene Griff proximalwärts bewegt, sodass der Einsatz von der Rastkugel proximalwärts weg bewegt wird und diese freigibt. Diese Lösebewegung ist sowohl beim Zusammensetzen als auch beim Trennen des Instrumenteneinsatzes von der Instrumentenhandhabe erforderlich. Insbesondere beim Zusammensetzen ist dieses Verriegelungssystem schwer zu handhaben, weil der Griff zum einen proximalwärts bewegt werden muss, um den Einsatz in seine gelöste Position zu bringen, und gleichzeitig die Instrumentenhandhabe mit dem Griff distalwärts auf das proximale Ende des Instrumenteneinsatzes zu bewegt werden muss, um beide Teile zusammenzufügen. Dies macht die Handhabung äußerst umständlich.

Ein weiteres Instrument entsprechend der vorangehenden Beschreibung ist aus WO 01/01 870 A1 bekannt.

Es ist daher Aufgabe der Erfindung, ein verbessertes medizinisches Instrument mit einem Instrumenteneinsatz und einer Instrumentenhandhabe, welche über eine Rastverbindung lösbar miteinander verbindbar sind, zu schaffen, bei welchem sich Instrumenteneinsatz und instrumentenhandhabe leichter zusammensetzen und trennen lassen.

Diese Aufgabe wird durch ein medizinisches Instrument mit den im Anspruch 1 angegebenen Merkmalen gelöst. Bevorzugte Ausführungsformen ergeben sich aus den Unteransprüchen.

Das erfindungsgemäße medizinische Instrument weist einen Instrumenteneinsatz sowie eine Instrumentenhandhabe auf, welche über eine Rastverbindung lösbar miteinander verbindbar sind. Die ermöglicht in bekannter Weise, das Instrument zur Reinigung zu zerlegen und verschiedene Instnirnenteneinsätze mit ein und derselbe Instrumentenhandhabe je nach Einsatzzweck zu kombinieren. Zur Verbindung von Instrumenteneinsatz und Instrumentenhandhabe sind zumindest ein Griffelement sowie ein Verschiusseiement zur Sicherung der Rastverbindung vorgesehen. Dabei können Griffelement und Verschlusselement entweder am distalen Ende der Instrumentenhandhabe oder am proximalen Ende des Instrumenteneinsatzes, angeordnet sein. Das Verschlusselement ist so angeordnet, dass es sich sowohl durch Bewegung des Griffelementes als auch unabhängig von einer Bewegung des Griffelementes in eine entsicherte Position bewegen lässt, um die Rastverbindung zu lösen. In diesem Zustand kann der Instrumenteneinsatz von der Instrumentenhandhabe getrennt werden oder mit der Instrumentenhandhabe verbunden werden. Erfindungsgemäß ist das Verschlusselement, weiches die Rastverbindung sichert, somit von dem Griffelement, welches zum Lösen der Rastverbindung bewegt werden muss, entkoppelt, Dies ermöglicht, dass beim Zusammensetzen von Instrumentenhandhabe und Instrumenteneinsatz durch Eingriff von korrespondierenden Bauteilen der Rastverbindung das Verschlusselement selbsttätig ohne Bewegung des Griffelements in die Löseposition bewegt wird. Es ist somit nicht mehr wie bei bislang bekannten Anordnungen erforderlich, beim Zusammensetzen auch das Griffelement in eine gelöste Position zu bewegen. Wenn beispielsweise Griffelement und Verschlusselement am distalen Ende der Instrumentenhandhabe angeordnet sind, ist es möglich, dass die Instrumentenhandhabe an dem Griffelement ergriffen wird und gemeinsam mit diesem distalwärts auf das proximale Ende des Instrumenteneinsatzes zu beweget wird, während gleichzeitig durch das proximale Ende des Instrumenteneinsatzes das Verschlusselement proximalwärts bewegt wird, ohne dass sich das Griffelement in dieser Richtung mitbewegen muss. Griffelement und Verschlusselement können somit beim Zusammensetzen entgegengesetzte Bewegungen ausführen. Dadurch wird ein wesentlich einfacheres Zusammensetzen von Instrumenteneinsatz und Instrumentenhandhabe erreicht.

Zum Lösen wiederum kann das Griffelement ergriffen werden und in eine Löserichtung bewegt werden, wobei bei Bewegung des Griffelementes das Verschlusselement mit in die gelöste Position bewegt wird, um die Rastverbindung freizugeben und den Instrumenteneinsatz von der Instrumentenhandhabe zu trennen. So kann bei dem genannten Beispiel das Griffelement proximalwärts bewegt werden und das Verschlusselement dadurch in proximaler Richtung mitbewegt werden, um die Rastverbindung freizugeben. Umgekehrt ist die Anordnung auch möglich, wenn Griffelement und Verschlusselement am distalen Ende des Instrumenteneinsatzes angeordnet sind. In diesem Fall wird die Verschlusshülse in die gelöste Position in distaler Richtung bewegt. Bei allen Anordnungen sind Griffelement und Verschlusselement derart entkoppelt, dass eine Bewegung des Griffelementes in die gelöste Position zwar gleichzeitig eine Bewegung des Verschlusselementes in die gelöste Position bewirkt, nicht jedoch eine Bewegung des Verschlusselementes gleichzeitig eine Bewegung des Griffelementes in die gelöste Position bedingt.

Die erfindungsgemäße Ausgestaltung hat somit den Vorteil, dass zum Zusammensetzen von Instrumentenhandhabe und Instrumenteneinsatz das Griffelement dazu verwendet wird, das mit ihm verbundene Bauteil, entweder die Instrumentenhandhabe oder den Instrumenteneinsatz zu ergreifen. Anschließend wird das Bauteil durch Bewegen des Griffelementes in Fügerichtung zu dem anderen Bauteil des Instrumentes bewegt, um die Bauteile, nämlich Instrumentenhandhabe und Instrumenteneinsatz zu verbinden. Umgekehrt wird zum Lösen das Griffelement ergriffen, um das mit ihm verbundene Bauteil von dem anderen Bauteil wegzubewegen. Durch die zum Trennen und Zusammenfügen von Instrumentenhandhabe und Instrumenteneinsatz ohnehin erforderliche Bewegung wird die Rastverbindung automatisch verriegelt bzw. gelöst. Die so geschaffene automatische Rastverbindung zwischen Instrumenteneinsatz und Instrumentenhandhabe verriegelt einerseits beim axialen Zusammenschieben von Instrumenteneinsatz und Instrumentenhandhabe durch Bewegung des Verschlusselementes automatisch die zusammengefügten Bauteile. Andererseits werden die selben beim Auseinanderziehen von Instrumenteneinsatz und Instrumentenhandhabe durch Bewegung des Griffelementes wieder automatisch entriegelt. Es müssen somit keine zusätzlichen Sicherungselemente manuell gelöst oder verriegelt werden.

Die Rastverbindung weist vorzugsweise zumindest ein bewegliches Rastelement auf, wobei das Verschlusselement in einer gesicherten Position das Rastelement blockiert und in einer entsicherten Position das Rastelement freigibt. Das Rastelement kann beispielsweise als Rastvorsprung oder Rastkörper ausgebildet sein, welcher in eine korrespondierende Ausnehmung eingreift, wobei das Verschlusselement das Rastelement so blockiert, dass es nicht aus der Ausnehmung heraus treten kann. In der gelösten Position ermöglicht das Verschlusselement eine Bewegung des Rastelementes, sodass beispielsweise ein Rastkörper oder ein Rastvorsprung von der Ausnehmung außer Eingriff tritt und die Rastverbindung gelöst, das heißt Instrumentenhandhabe und Instrumenteneinsatz voneinander getrennt werden können. Die Rastelemente sind vorzugsweise in radialer Richtung beweglich, um mit korrespondierenden Rastelementen, beispielsweise Rastausnehmungen, in und außer Eingriff treten zu können. Das Verschlusselement, welches die Rastelemente sichert, ist vorzugsweise in axialer Richtung beweglich, um die Rastverbindung zu sichern und zu lösen. Auf das Verschlusselement wird entsprechend eine axial gerichtete Verriegelungskraft aufgebracht. Die Kombination von axial gerichteter Verriegelungskraft und radial beweglichen Rastelementen ermöglicht eine besonders kompakte Ausgestaltung der Rastverbindung im Verglich zu einer Anordnung, bei welcher auch die Verriegelungskraft in radialer Richtung aufgebracht wird.

Weiter bevorzugt ist das Rastelement eine Rastkugel. Derartige Rastkugeln ermöglichen ein leichtes Zusammensetzen, da sie leicht in korrespondierende Rastausnehmungen hineingleiten.

Alternativ kann das Rastelement als federnde Rastzunge ausgebildet sein. Derartige Rastelemente haben den Vorteil, dass sie sich kostengünstig als Spritzgussteile aus Kunststoff fertigen lassen. Am freien Ende der Rastzungen sind Rastvorsprünge ausgebildet, welche in korrespondierende Rastöffnungen eingreifen oder korrespondierende Rastvorsprünge hintergreifen können.

Vorzugsweise greifen die Rastelemente in eine korrespondierende ringförmige Rastnut ein. Dies ermöglicht eine drehbare Ausgestaltung der Rastverbindung, bei welcher beim Zusammensetzen von Instrumenteneinsatz und Instrumentenhandhabe nicht auf eine bestimmte Winkellage geachtet werden muss, da die Rastelemente in jeder Winkellage in die ringförmige Rastnut eingreifen können. Ferner ermöglicht eine solche Rastverbindung, dass auch im verbunden Zustand die Rastelemente in der ringförmigen Rastnut gedreht werden können, sodass beispielsweise der Instrumenteneinsatz gegenüber der Instrumentenhandhabe um seine Längsachse gedreht werden kann.

Vorzugsweise sind das Griffelement und das Verschlusselement in Richtung der Längsachse des Instrumenteneinsatzes bewegbar. Diese Anordnung ermöglicht eine schlanke Ausgestaltung der Rastverbindung zwischen Instrumentenhandhabe und Instrumenteneinsatz. Darüber hinaus wird ein einfaches Zusammensetzen und Trennen von Instrumenteneinsatz und Instrumentenhandhabe möglich, da das Griffelement und das Verbindungselement zum Lösen bzw. Verriegeln in der Bewegungsrichtung des Instrumenteneinsatzes und der Instrumentenhandhabe bewegt werden.

Das Verschlusselement wird zweckmäßigerweise durch Federkraft in der gesicherten Position gehalten. Dadurch kann eine selbsttätige Sicherung beim Zusammensetzen von Instrumenteneinsatz und Instrumentenhandhabe erreicht werden. Ferner kann ein unbeabsichtigtes Lösen bei Benutzung des Instrumentes verhindert werden. Die Federkraft kann beispielsweise durch eine axial wirkende Schraubenfeder aufgebracht werden.

Das Verschlusselement ist vorzugsweise als Verschlusshülse und das Griffteil als Griffhülse ausgebildet, welche in Richtung der Längsachse des Instrumenteneinsatzes beweglich geführt sind, wobei die Verschlusshülse im Inneren der Griffhülse angeordnet ist. Diese Anordnung ermöglicht zum einen eine rotationssymmetrische Ausgestaltung der Rastverbindung, wodurch eine Drehbarkeit des Instrumenteneinsatzes gegenüber der Instrumentenhandhabe erreicht werden kann. Darüber hinaus kann eine kompakte Ausgestaltung der Rastverbindung erreicht werden, welche eine insgesamt schlanke Gestalt des Instrumentes ohne störende Vorsprünge oder vorstehende Betätigungsmittel ermöglicht. Die Anordnung der Verschlusshülse im Inneren der Griffhülse hat den Vorteil, dass die Verschlusshülse nach außen durch die Griffhülse abgedeckt wird, sodass die Bewegung der Verschlusshülse bei der Handhabung des Instrumentes nicht versehentlich blockiert werden kann. Darüber hinaus wird eine Verletzungsgefahr beispielsweise durch Einklemmen bei der Bewegung der Verschlusshülse verhindert.

Weiter bevorzugt weist die Verschlusshülse zumindest eine Anlageschulter auf, welche von einer an der Griffhülse ausgebildeten Anlageschulter lediglich an einer in Bewegungsrichtung zu der entsicherten Position hin rückwärtigen Fläche hintergriffen wird. Diese Ausgestaltung bewirkt, dass bei Bewegung der Griffhülse zu der entsicherten Position hin, die Anlageschulter der Griffhülse an der in Bewegungsrichtung rückwärtigen Fläche der Anlageschulter der Verschlusshülse zur Anlage kommt und somit als Mitnehmer die Verschlusshülse ebenballs in Richtung der entsicherten Position drückt bzw. schiebt. Umgekehrt drückt, wenn die Verschlusshülse in Richtung der gesicherten Position bewegt wird und sich die Griffhülse zuvor ebenfalls in der entsicherten Position befand, die Anlageschulter der Verschlusshülse gegen die Anlageschulter der Griffhülse und schiebt diese ebenfalls in Richtung der gesicherten Position. Dies ist insbesondere dann von Vorteil, wenn ein Federelement vorgesehen ist, welches die Verschlusshülse in die gesicherte Position drückt, da das Federelement so auch die Griffhülse in der gesicherten Position hält. Da jedoch die Anlageschultern in der entgegengesetzten Richtung einander nicht hintergreifen, kann die Verschlusshülse in die entsicherte Position bewegt werden, ohne die Griffhülse mitzunehmen, das heißt die Griffhülse kann in Ihrer Ausgangslage in der gesicherten Position verbleiben. Die Anlageschultern von Griffhülse und Verschlusshülse sind vorzugsweise so ausgebildet, dass sich die Anlageschulter der Verschlusshülse radial nach außen erstreckt, während die Anlageschulter der Griffhülse am Innenumfang der Griffhülse ausgebildet ist. Idealerweises sind beide Schultern ringförmig und rotationssymmetrisch ausgebildet, sodass eine kostengünstig Fertigung und eine einfache Montage des Instrumentes erreicht werden kann, da nicht auf vorbestimmte Winkellagen der einzelnen Bauteile zueinander geachtet werden muss.

Gemäß einer besonderen Ausführungsform, welche nicht auf eine der vorangehend beschriebenen Ausgestaltungen beschränkt ist, weist die Rastverbindung ein Rastelement auf, welches zur Verbindung von Instrumenteneinsatz und Instrumentenhandhabe in eine korrespondierende Rastnut eingreift, welche axial einen gewellten oder gezackten Rand aufweist. Eine solche Ausgestaltung der Rastverbindung ermöglicht, dass das Rastelement in der Rastnut gedreht werden kann, wobei durch die Wellen oder Zacken am Rand eine Positionierung erreicht wird. So kann der Instrumenteneinsatz gegenüber der Instrumentenhandhabe rastend verdreht werden, wobei zwischen den einzelnen durch die Wellen oder Zacken vorgegebenen Rastpositionen leichte Widerstände beim Verdrehen zu überwinden sind. Dazu kann ein Federelement vorgesehen sein, um das Rastelement federnd in die Wellen oder Zacken des Randes einzudrücken und so eine Positionierung beim Verdrehen zu gewährleisten. Beim Einsatz in einer medizinischen Zange ermöglicht diese Ausgestaltung ferner, dass, wenn auf die Zangenhandhabe eine Kraft aufgebracht wird, über die Betätigungsstange und das Zangenmaul eine Kraft auf den Schaft des Instrumenteneinsatzes wirkt, durch die das Rastelement gegen den gewellten oder gezackten Rand gedrückt wird, sodass es in den Vertiefungen des Randes gehalten wird. Dadurch wird sichergestellt, dass bei Betätigung des Instrumentes eine Verdrehung nicht mehr möglich ist, sondern der Instrumenteneinsatz in einer zuvor eingestellten Winkelposition bezüglich der Instrumentenhandhabe gehalten wird. Diese Ausgestaltung kann auch unabhängig von den oben beschriebenen Merkmalen, das heißt insbesondere unabhängig von der Ausgestaltung von Griffelement und Verschlusshülse eingesetzt werden.

Vorzugsweise sind das Griffelement und das Verschlusselement am proximalen Ende des Instrumenteneinsatzes angeordnet. Alternativ kann die Erfindung jedoch auch in der Weise verwirklicht werden, dass Griffelement und Verschlusselement am distalen Ende der Instrumentenhandhabe angeordnet sind.

Besonders bevorzugt ist das Griffelement drehfest mit dem Instrumenteneinsatz verbunden. Hierzu kann eine entsprechende Linearführung zwischen Griffelement und Instrumentenschaft des Instrumenteneinsatzes vorgesehen sein. Dies ermöglicht, dass durch Ergreifen des Griffelementes der Instrumenteneinsatz gedreht werden kann, beispielsweise um den Instrumenteneinsatz in einer vorbestimmten Winkellage bezüglich seiner Längsachse mit der Instrumentenhandhabe zu verbinden. Für den Fall, dass die Verbindung zwischen Instrumenteneinsatz und Instrumentenhandhabe drehbar ausgebildet ist, kann ferner der Instrumenteneinsatz an dem Griffelement ergriffen werden, um ihn gegenüber der Instrumentenhandhabe zu verdrehen.

Gemäß einer weiteren besonderen Ausführungsform, welche auch unabhängig von den vorangehend beschriebenen Ausführungsformen verwirklicht werden kann, weist der Instrumenteneinsatz ein Schaftrohr, eine im Inneren des Schaftrohres angeordnete Betätigungsstange und zumindest ein am distalen Ende angeordnetes über die Betätigungsstange bewegliches Element auf. Dieses bewegliche Element kann beispielsweise ein Maulteil eines Zangenmaules, welches am distalen Ende des Instrumenteneinsatzes ausgebildet ist, sein. Gemäß dieser besonderen Ausführungsform ist im Kraftfluss zur Bewegung des beweglichen Elementes zumindest ein Federelement als Überlastsicherung angeordnet. Das Federelement bewirkt, dass in dem Fall, dass die auf das bewegliche Element, beispielsweise ein Zangenmaul, wirkende Betätigungskraft die Federkraft übersteigt, die Feder gestaucht wird, sodass keine größere Kraft auf das bewegliche Element bzw. das Zangenmaul aufgebracht werden kann. Dadurch kann zum einen eine Beschädigung des Instrumentes verhindert werden, zum anderen kann das Instrument so ausgestaltet werden, dass beispielsweise Gewebeteile mit einer voreingestellten Maximalkraft ergriffen werden können. Dadurch, dass das Federelement mit einer definierten Kraft vorgespannt ist, ist es bis zu einem Erreichen dieser Kraft beim Betätigen des Instrumentes über den gesamten Bewegungsraum überhaupt nicht spürbar. Eine feinfühlige Betätigung des Instrumentes ist weiter möglich. Die Federkonstante des Federelementes ist vorzugsweise so ausgelegt, dass sich die Vorspannkraft bei Verformung des Federelementes um den Weg, den die Betätigungsstange des Instrumentes maximal ausführen kann, um nicht mehr als 20 bis 50 % erhöht. Vorzugsweise ist das Federelement eine Druckfeder, welche sich in Richtung der Längsachse des Instrumenteneinsatzes erstreckt. Die Anordnung dieses Überlastschutzes kann auch unabhängig von den vorangehend beschriebenen Merkmalen verwirklicht werden, insbesondere kann ein solcher Überlastschutz auch bei Instrumenten eingesetzt werden, welche keine Rastverbindung zwischen Instrumenteneinsatz und Instrumentenhandhabe gemäß der vorangehend beschriebenen Ausgestaltung aufweisen.

Weiter bevorzugt ist das Schaftrohr des Instrumenteneinsatzes an seinem proximalen Ende in einem Verbindungselement zur Verbindung mit einer Instrumentenhandhabe gelagert und stützt sich in proximaler Richtung an dem Verbindungselement über ein axial wirkendes Federelement ab. Ein solches Federelement ist vorzugsweise eine Druckfeder in Form einer Schraubenfeder. Wenn bei dieser Ausgestaltung eine Betätigungstange im Inneren des Schaftrohres proximalwärts bewegt wird, wird bei geschlossenem Zangenmaul ebenfalls eine in proximaler Richtung wirkende Kraft auf das Schaftrohr aufgebracht. Diese Kraft wird über das axial wirkende Federelement auf das Verbindungselement zu der Instrumentenhandhabe übertragen. Überschreitet nun die auf das Schaftrohr aufgebrachte Betätigungskraft die Federkraft des Federelementes, so wird sich dieses verformen, sodass die maximal übertragbare Kraft durch die Federkraft des Federelementes begrenzt wird. Auf diese Weise können die empfindlichen Gelenke und Hebel eines Zangenmauls vor Überlast geschützt werden.

Nachfolgend wir die Erfindung beispielhaft anhand der beigefügten Figuren beschrieben. In diesen zeigt:
- Fig. 1: eine Schnittansicht einer erfindungsgemäßen Rastverbindung zwischen Instrumenteneinsatz und Instrumentenhandhabe gemäß einer ersten Ausführungsform,
- Fig. 2: eine Schnittansicht einer Rastverbindung zwischen Instrumentenhandhabe und Instrumenteneinsatz gemäß einer zweiten Ausführungsform während des Zusammensetzens,
- Fig. 3: die Rastverbindung gemäß Fig. 2 im verriegelten Zustand,
- Fig. 4: die Rastverbindung gemäß Fig. 2 und 3 beim Trennen von Instrumenteneinsatz und Instrumentenhandhabe, und
- Fig. 5: schematisch eine Außenansicht des proximalen Ende des Instrumenteneinsatzes gemäß Fig. 2 bis 4.

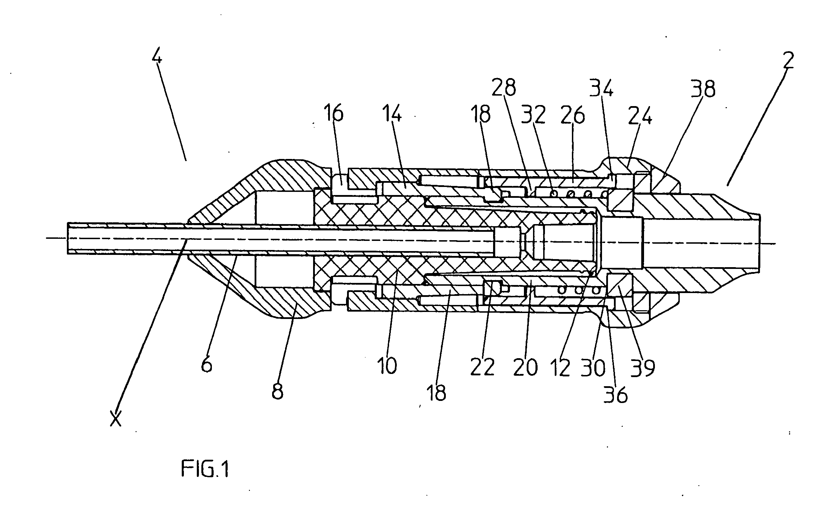

Figur 1 zeigt eine Schnittansicht der Rastverbindung zwischen einer Instrumentenhandhabe 2 und einem Instrumenteneinsatz 4, wobei in Figur 1 lediglich das distale Ende der Instrumentenhandhabe 2 sowie das proximale Ende des Instrumenteneinsatzes 4 dargestellt sind. Der Instrumenteneinsatz 4 weist ein Schaftrohr 6 auf, welches sich am proximalen Ende des Instrumenteneinsatzes in ein Gehäuseteil 8 erstreckt und an seinem proximalen Ende in ein Anschlussteil 10 eingreift und in diesem fixiert ist. Das proximale Ende des Anschlussteiles 10 ist als Lueranschluss 12 ausgebildet, um einen leichten Anschluss vom Spülleitungen zum Reinigen des Schaftrohres 6 zu ermöglichen.

In den Gehäuseteil 8 ist vom proximalen Ende her ein Verbindungsteil 14 eingesetzt, welches über Schnapphaken 16 in dem Gehäuseteil 8 fixiert ist. Das Verbindungsteil 14 ist so ausgebildet, dass es neben den am distalen Ende angeordneten Schnapphaken 16 Schnapphaken 18 aufweist, welche sich in proximaler Richtung erstrecken und an ihrem proximalen freien Enden radial nach innen gerichtete Verdickungen in Form von Rastvorsprüngen aufweisen.

Die Instrumentenhandhabe 2 weist einen sich distalwärts erstreckenden Gehäuseabschnitt 20 auf, in welchem am Außenumfang eine ringförmige Rastnut 22 ausgebildet ist. In diese Rastnut 22 greifen die verdickten Abschnitte der Rastzungen 18 zur Verbindung von Instrumentenhandhabe 2 und Instrumenteneinsatz 4 ein.

Am Außenumfang des Gehäuseabschnittes 20 ist eine Griffhülse 24 axial in Richtung der Längsachse X beweglich geführt. Im Inneren der Griffhülse 24 ist konzentrisch zu dieser eine Verschlusshülse 26 angeordnet, welche ebenfalls axial in Richtung der Längsachse X beweglich auf dem Außenumfang des Gehäuseabschnittes 20 geführt ist. Die Verschlusshülse 26 ist an ihrem distalen Ende so ausgebildet, dass sie in einer Verriegelungsposition bzw. gesicherten Position die distalen Enden der Rastzungen 18 umfänglich übergreift, sodass sich die Rastzungen 18 nicht radial nach außen bewegen können und die Verdickungen der Rastzungen 18 in dieser Position fest in Eingriff mit der Rastnut 22 gehalten werden. Am Innenumfang der Verschlusshülse 26 ist eine ringförmige Anlageschulter 28 ausgebildet. Ferner ist am Außenumfang des Gehäuseabschnittes 20 eine distalwärts gerichtete Anlageschulter 30 vorgesehen. Die Anlageschulter 28 und die Anlageschulter 30 sind in Richtung der Längsachse X voneinander beabstandet und zwischen den beiden Anlageschultern 28 und 30 ist eine Druckfeder 32 angeordnet. Die Druckfeder 32 ist als Schraubenfeder ausgebildet und konzentrisch zur Längsachse X angeordnet. Die Druckfeder 32 drückt die Verschlusshülse 26 über die Anlageschulter 28 in ihre gesicherte Position. Auf diese Weise wird sichergestellt, dass die Verschlusshülse 26 selbsttätig in die gesicherte Position bewegt und in dieser gehalten wird, sodass die Rastzungen 18 nicht von der Rastnut 22 außer Eingriff treten können.

Am proximalen Ende weist die Verschlusshülse 26 einen radial nach außen gerichteten Vorsprung 34 auf. Die Griffhülse 24 ist an ihrem proximalen Ende am Innenumfang radial erweitert, sodass eine proximalwärts gerichtete Anlageschulter 36 gebildet wird. Ein Führungselement 38, welches am Außenumfang des Gehäuseabschnittes 20 in Richtung der Längsachse X beweglich geführt ist, verschließt die Griffhülse 24 an deren proximalen Ende. Ferner hintergreift das Führungselement 38 den die Anlageschulter 30 bildenden ringförmigen Vorsprung 39 an der proximalen Seite. Der Vorsprung 38 begrenzt so die Bewegung der Griffhülse 24 in distaler Richtung.

Der Vorsprung 34 liegt mit seiner distalwärts gerichteten Seitenfläche an der Anlageschulter 36 im Inneren der Griffhülse 24 an. Dies bewirkt, dass über die Verschlusshülse 26 auch die Griffhülse 24 von der Druckfeder 32 in die gezeigte gesicherte Position gedrückt wird. Zum Lösen der Rastverbindung wird die Griffhülse 24 ergriffen und proximalwärts bewegt, wodurch über die Anlageschulter 26 und den Vorsprung 34 die Verschlusshülse 26 mit in proximaler Richtung gegen die Federkraft der Druckfeder 32 bewegt wird. Dabei kommt das distale Ende der Verschlusshülse 26 von dem Rastzungen 18 außer Eingriff, sodass diese sich radial nach außen bewegen können. Dabei treten die Verdickungen der Rastzungen 18 radial aus der Rastnut 22 heraus, sodass der Instrumenteneinsatz 4 von der Instrumentenhandhabe 2 getrennt werden kann. Wenn nun das Griffteil 24 losgelassen wird, werden Verschlusshülse 26 und Griffteil 24 über die Druckfeder 32 selbsttätig wieder in die ursprüngliche Verriegelungsposition bewegt.

Wenn die Instrumentenhandhabe 2 und der Instrumenteneinsatz 4 wieder zusammengesetzt werden sollen, können die Instrumentenhandhabe 2 an der Griffhülse 24 und der Instrumenteneinsatz 4 an dem Gehäuseteil 8 ergriffen werden und der Instrumenteneinsatz 4 und die Instrumentenhandhabe 2 können in Richtung der Längsachse X aufeinander zu bewegt werden. Wenn der Instrumenteneinsatz 4 mit dem Anschlussteil 10 in den Gehäuseabschnitt 20 des Instrumenteneinsatzes eingesetzt wird, kommen die proximalen Stirnkanten der Rastzungen 18 zur Anlage an der distalen Stirnfläche der Verschlusshülse 26. Bei weiterer Bewegung des Instrumenteneinsatzes 4 auf die Instrumentenhandhabe 2 zu, wird dann über die Rastzungen 18 die Verschlusshülse 26 gegen die Federkraft der Feder 32 proximalwärts verschoben. Dabei kommt der Vorsprung 34 von der Anlageschulter 36 der Griffhülse 24 außer Eingriff, sodass die Griffhülse in ihrer ursprünglichen gesicherten Position verbleibt, während die Verschlusshülse soweit proximalwärts verschoben wird, das die Rastzunge 18 wieder in die Rastnut 32 eintreten können. Dabei bewegen sich die Verdickungen an den freien Enden der Rastzungen 18 aufgrund deren Elastizität selbsttätig in die Rastnut 20 radial nach innen, sodass die Rastzungen 18 in das Innere der Verschlusshülse 26 eintreten können. Dabei kommt die distale Stirnfläche der Verschlusshülse 26 von den Rastzungen 18 außer Eingriff und die Verschlusshülse 26 wird von der Druckfeder 32 selbsttätig in die gesicherte Position gedrückt wird, in welcher die Rastzungen 18, wie gezeigt, umfänglich von der Verschlusshülse 26 umgriffen und so in der Rastnut 20 gesichert werden. Beim Zusammensetzen besteht somit erfindungsgemäß der Vorteil, dass die Griffhülse 24 zum Verriegeln der Rastverbindung nicht entgegen der Bewegungsrichtung beim Zusammensetzen bewegt werden muss, sondern in ihrer ursprünglichen Lage verbleiben kann. Dadurch wird das Zusammensetzen des Instrumentes erheblich vereinfacht.

Auch wenn bei dem Beispiel gemäß Fig. 1 Griffhülse und Verschlusshülse am distalen Ende der Instrumentenhandhabe 2 angeordnet sind, kann die Erfindung auch in der Weise verwirklicht werden, dass Griffund Verschlusshülse am proximalen Ende des Instrumenteneinsatzes vorgesehen werden.

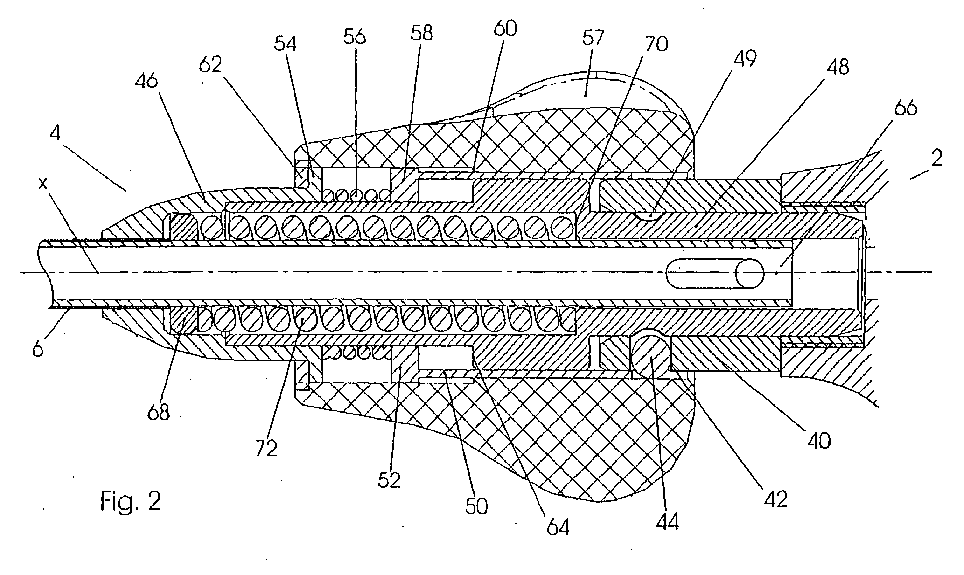

Eine solch umgekehrte Anordnung ist bei der zweiten anhand von Fig. 2 bis 5 beschriebenen Ausführungsform gegeben. Fig. 2 zeigt eine Schnittansicht der Rastverbindung zwischen Instrumentenhandhabe 2 und Instrumenteneinsatz 4. Ein weiterer Unterschied zu der Ausführungsform gemäß Fig. 1 liegt darin, dass an Stelle von Rastzungen 18 eine Kugelrastverbindung vorgesehen ist. Von der Instrumentenhandhabe 2 erstreckt sich ein hülsenförmiger Gehäuseabschnitt 40 distalwärts. In dem Gehäuseabschnitt 40 sind eine oder mehrere sich radial erstreckende Bohrungen 42 ausgebildet, in denen Rastkugeln 44 angeordnet sind. Die Rastkugeln 44 weisen einen Durchmesser auf, welcher größer als die radiale Erstreckung der Bohrungen 42 bzw. der Wandstärke des Gehäuseabschnittes 40 ist.

Der Instrumenteneinsatz 4 weist ein Schaftrohr 6 auf, welches an seinem proximalen Ende in ein Gehäuseteil 46 eintritt. Das Gehäuseteil 46 geht an seinem proximalen Ende in ein fest verbundenes Verbindungsteil 48 über. In einem proximalen Abschnitt des Verbindungsteils 48 ist an dessen Außenumfang eine ringförmige Rastnut 49 ausgebildet, in welche die Rastkugeln 44 verriegelnd eingreifen können. Wenn die Rastkugeln 44 in die Rastnut 49 eingreifen, sind die Instrumentenhandhabe 2 sowie der Instrumenteneinsatz 4 fest miteinander verbunden. Zur Sicherung dieser Rastverbindung ist eine Verschlusshülse 50 vorgesehen, welche auf dem Außenumfang des Verbindungsteils 48 in Längsrichtung X beweglich geführt ist. In einer Verriegelungsposition überdeckt das proximale Ende der Verschlusshülse 50 die Bohrungen 42, sodass die Rastkugeln 44 in Eingriff mit der Rastnut 49 gehalten werden. Wenn die Verschlusshülse 50 in die gezeigte gelöste bzw. entsicherte Position bewegt wird, gibt das proximale Ende der Verschlusshülse 50 die Bohrungen 42 frei, sodass sich die Rastkugeln 44 radial nach außen bewegen können, wenn der Instrumenteneinsatz 4 aus der Instrumentenhandhabe 2 entnommen werden soll.

Am distalen Ende weist die Verschlusshülse 50 eine radial nach innen gerichteten Vorsprung auf, welcher ebenfalls gleitend am Außenumfang des Verbindungsteils 48 geführt ist. Der Gehäuseteil 46 weist an seinem proximalen Ende eine radial nach außen gerichtete Auskragung 54 auf. Zwischen der Auskragung 54 und dem Vorsprung 52 der Verschlusshülse 50 ist eine Druckfeder 56 in Form einer Schraubenfeder parallel zur Längsachse X angeordnet. Die Druckfeder 56 drückt die Verschlusshülse 52 proximalwärts in die verriegelte Position, in der die Bohrungen 42 am Außenumfang von der Verschlusshülse 50 überdeckt werden.

Die Verschlusshülse 50 ist von einer Griffhülse 57 umgeben, welche am Außenumfang des Gehäuseteils 46 sowie der Verschlusshülse 50 in Längsrichtung X beweglich geführt ist. Die Verschlusshülse 50 weist an der Position des Vorsprungs 52 ferner eine nach außen gerichtete Auskragung 58 auf, welche am Innenumfang der Griffhülse 57 anliegt. Am Innenumfang der Griffhülse 57 ist eine distalwärts gerichtete Anlageschulter 60 ausgebildet, welche als Anschlag für die Auskragung 58 der Verschlusshülse 50 dient. Wenn die Verschlusshülse 50 über die Druckfeder 56 proximalwärts gedrückt wird, kommt die Auskragung 58 an der Anlageschulter 60 zur Anlage, wodurch die Griffhülse 57 ebenfalls von der Druckfeder 56 in eine verriegelte Position gedrückt wird und in dieser gehalten wird. Als Anschlag weist die Griffhülse 57 dabei an ihrem distalen Ende eine radial nach innen gerichtete Auskragung 62 auf, welche an der distalen Seite der Auskragung 54 des Gehäuseteils 46 zur Anlage kommt und somit die Bewegung der Griffhülse 57 in proximaler Richtung begrenzt.

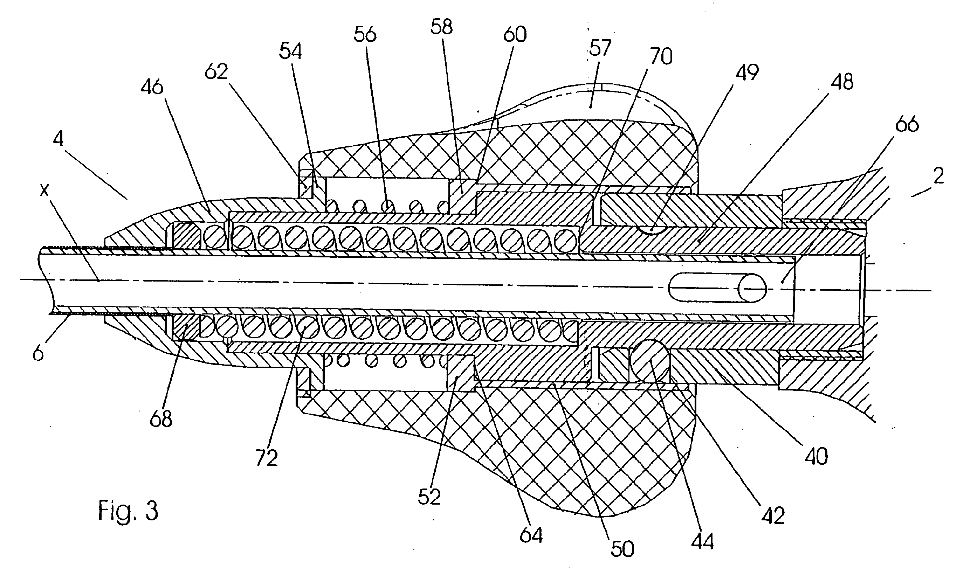

Auch die Anordnung gemäß Fig. 2 ermöglicht die entkoppelte Bewegung von Verschlusshülse 50 und Griffhülse 57. Wenn Instrumentenhandhabe 2 und Instrumenteneinsatz 4 zusammengesteckt werden, gleiten die Rastkugeln 44 zunächst auf dem Außenumfang des Verbindungsteils 48, wobei sie radial nach außen aus den Bohrungen 42 hervorragen. Dabei kommen die Raskugeln 42 mit der proximalen Stirnseite der Verschlusshülse 50 in Kontakt und schieben diese gegen die Federkraft der Druckfeder 56 in distaler Richtung in eine entriegelte Position. Dabei kommt die Auskragung 58 von der Anlageschulter 60 der Griffhülse 57 außer Eingriff. Die Griffhülse 57 kann somit in ihrer ursprünglichen Lage verbleiben und der an der Griffhülse 57 ergriffene Instrumenteinsatz 4 kann problemlos weiter proximalwärts auf die Instrumentenhandhabe 2 zu bewegt werden. Wenn die Rastkugeln 44 bei der Bewegung in Richtung der Längsachse X die Position der Rastnut 49 erreichen, können die Rastkugeln 44 in die Rastnut 49 eintreten, wobei die Verschlusshülse 50 durch die Druckfeder 56 proximalwärts bewegt wird und dabei die Rastkugeln 44 in die Rastnut 50 drückt. Die Bewegung der Verschlusshülse 50 in proximaler Richtung wird durch eine Stufe 64 am Außenumfang des Verbindungsteils 48 begrenzt. Wenn der Vorsprung 52 an der Stufe 64 anliegt, hat die Verschlusshülse 50 die in Fig. 3 gezeigte verriegelte Position erreicht, in der sie die Bohrungen 52 überdeckt und die Rastkugeln 44 in der Rastnut 49 sichert. Gleichzeitig liegt die Auskragung 58 an der Anlageschulter 60 an und hält somit die Griffhülse 57 ebenfalls in ihrer proximalen Position.

Die in Fig. 2 gezeigte Ausführungsform weist darüber hinaus eine Überlastsicherung für die Betätigung eines beweglichen Elementes am distalen Ende des Instrumenteneinsatzes 4 auf. Im Inneren des Schaftrohres 6 ist eine Betätigungsstange 66 angeordnet, welche proximalwärts bewegt werden kann, beispielsweise um ein Zangenmaul am distalen Ende des Instrumenteinsatzes 4 zu schließen. Das proximale Ende des Schaftrohres 6 weist ein radial nach außen auskragendes Ringelement 68 auf. Zwischen der proximalen Seite des Ringelementes 68 und einer distalwärts gerichteten Anlageschulter 70 im Inneren des Verbindungsteils 48 ist eine Druckfeder 42 angeordnet, welche als Übedastschutz fungiert. Wenn die Betätigungsstange 66 proximalwärts bewegt wird, um ein Zangenmaul am distalen Ende des Schaftrohres 6 zu schließen, wird über das geschlossene Zangenmaul und gegebenenfalls über ein von diesem ergriffenes Element, beispielsweise ein Gewebeteil, eine Kraft auf das Schaftrohr 6 in proximaler Richtung aufgebracht. Diese Kraft wird am proximalen Ende des. Schaftrohres 6 über das Ringelement 68 auf die Druckfeder 72 und von dieser auf das Verbindungsteil 48 und über dieses weiter zu der Instrumentenhandhabe 2 übertragen. Wenn nun die zu übertragende Kraft die Federkraft der Druckfeder 72 überschreitet, wird diese gestaucht, und somit die übertragbare Kraft begrenzt. Auf diese Weise können empfindliche Element Gelenke oder Hebel des Zangenmauls vor Überlast geschützt werden. Die Federkonstante der Druckfeder 72 ist vorzugsweise so gewählt, dass sich bei der im Instrument maximal möglichen Stauchung die Federkraft um nicht mehr als 20 bis 50 % erhöht. Die so ausgestaltete Überlastsicherung kann auch bei anderen Instrumenten verwirklicht werden, welche nicht die beschriebenen Rastverbindungen aufweisen.

Fig. 3 zeigt den vollständig verriegelten Zustand der Rastverbindung gemäß Fig. 2. Die Verschlusshülse 50 überdeckt die Bohrungen 42, sodass die Rastkugeln 44 sicher in der Rastnut 49 gehalten werden. Wie zuvor beschrieben hält die Druckfeder 56 die Verschlusshülse 50 und die Griffhülse 57 in dieser gesicherten Position. Im Übrigen wird auf die vorangehende Beschreibung verwiesen.

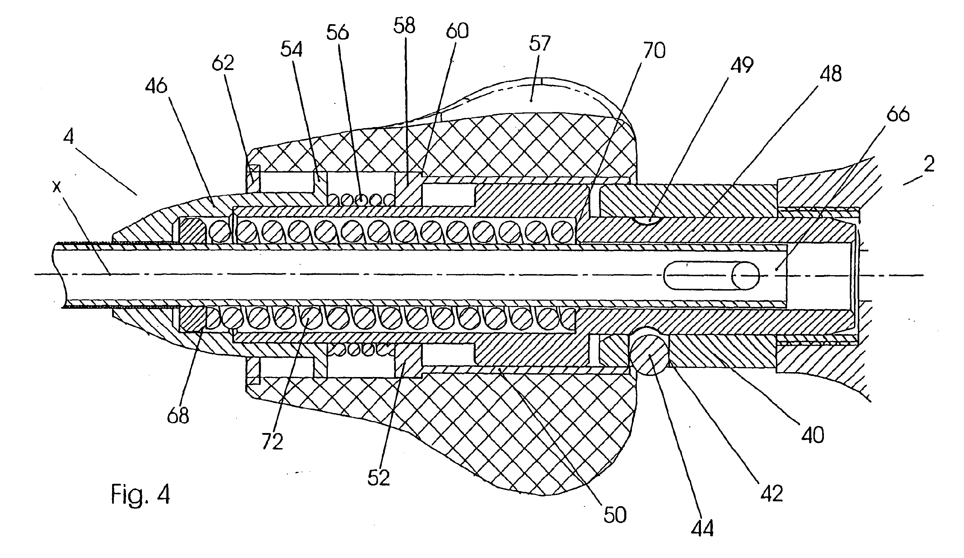

Fig. 4 zeigt, wie die Rastverbindung gemäß Fig. 2 und 3 gelöst wird. Ausgehend von dem in Fig. 3 gezeigten gesicherten Zustand werden die Instrumentenhandhabe 2 und die Griffhülse 57 des Instrumenteneinsatzes 4 ergriffen und auseinander gezogen. Dabei wird die Griffhülse 57 distalwärts bewegt und nimmt über die Anlageschulter 60 und die Auskragung 58 die Verschlusshülse 50 mit, sodass die Verschlusshülse 50 von den Bohrungen 42 wegbewegt wird und die Rastkugeln 44 freigibt, sodass diese sich radial nach außen bewegen können. So kommen die Rastkugeln 44 von der Rastnut 49 außer Eingriff und der Instrumenteneinsatz 4 kann aus der Instrumentenhandhabe 2 herausgezogen werden.

Die Griffhülse 57 fungiert gleichzeitig als Drehrad und ist dazu drehfest auf dem Gehäuseteil 46 des Instrumenteneinsatzes geführt. Dies ermöglicht, dass der der gesamte Instrumenteneinsatz durch Drehung der Griffhülse 57 um die Längsachse X bezüglich der Instrumentenhandhabe 2 gedreht werden kann. Dabei können sich die Rastkugeln 44 in der ringförmigen Rastnut 49 drehen.

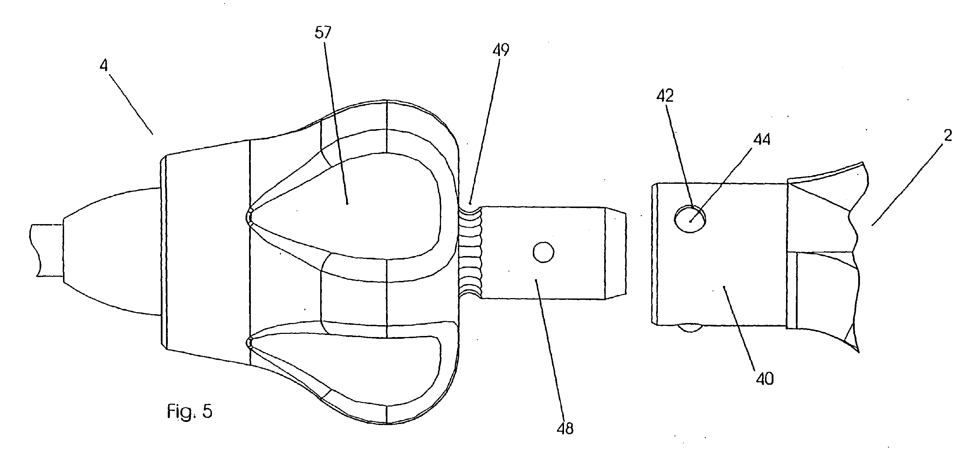

Fig. 5 zeigt eine nicht geschnittene Ansicht des proximalen Endes des Instrumenteneinsatzes 4 sowie des distalen Endes der Instrumentenhandhabe 2 gemäß der in Fig. 2 bis 4 dargestellten Ausführungsformen. Wie in Fig. 5 zu erkennen ist, ist die Rastnut 49 gewellt ausgebildet, das heißt sie hat insbesondere in axialer Richtung einen gewellten bzw. gezackten Rand. Dies bewirkt, dass bei geringfügiger Belastung beim Drehen des Drehrades, bzw. der Griffhülse 57 relativ zu der Instrumentenhandhabe 2 radiale Rastpositionen spürbar sind, sodass der Instrumenteneinsatz 4 rastend gegenüber der Instrumentenhandhabe 2 verdreht werden kann. Wird die Belastung stärker, wenn beispielsweise ein Maulteil am distalen Ende des Instrumenteneinsatzes 4 über die Betätigungsstange 66 geschlossen wird, werden die Rastkugeln 44 in den Wellentälern bzw. Vertiefungen des gezackten Randes der Rastnut 49 festgehalten, sodass sie sich nicht mehr umfänglich in der Nut 49 bewegen lassen. Damit ist die Rotation des Instrumenteneinsatzes 4 bei Betätigung der Betätigungsstange 6, das heißt beispielsweise beim Greifen, blockiert. Diese Ausgestaltung der Rastnut 49 kann auch unabhängig von der vorangehend beschriebenen Ausgestaltung mit Verschlusshülse 50 und Griffhülse 57 zum Einsatz kommen.

Auch bei der Ausführungsform gemäß Fig. 2 bis 5, könnten Griffhülse 57 und Verschlusshülse 50 in entsprechender Ausgestaltung an der Instrumentenhandhabe 2 angeordnet werden.

### Bezugszeichenliste

- 2: Instrumentenhandhabe
- 4: Instrumenteneinsatz
- 6: Schaftrohr
- 8: Gehäuseteil
- 10: Anschlussteil
- 12: Lüranschluss
- 14: Verbindungsteil
- 16: Schnapphaken
- 18: Rastzungen
- 20: Gehäuseabschnitt
- 22: Rastnut
- 24: Griffhülse
- 26: Verschlusshülse
- 28: Anlageschulter
- 30: Anlageschulter
- 32: Druckfeder
- 34: Vorsprung
- 36: Anlageschulter
- 38: Führungselement
- 39: Vorsprung
- 40: Gehäuseabschnitt
- 42: Bohrung
- 44: Rastkugeln
- 46: Gehäuseteil
- 48: Verbindungsteil
- 49: Rastnut
- 50: Verschlusshülse
- 52: Vorsprung
- 54: Auskragung
- 56: Druckfeder
- 57: Griffhülse
- 58: Auskragung
- 60: Anlageschulter
- 62: Auskragung
- 64: Stufe
- 66: Betätigungsstange
- 68: Ringelement
- 70: Anlageschulter
- 72: Druckfeder
- X: Längsachse

## Patentansprüche

1. Medizinisches Instrument mit einem Instrumenteneinsatz (4) und einer Instrumentenhandhabe (2), welche über eine Rastverbindung lösbar miteinander verbindbar sind, wobei ein Verschlusselement (26; 50) zur Sicherung der Rastverbindung sowie ein Griffelement (24; 57), weiches zum Lösen der Rastverbindung bewegt werden muss, vorgesehen sind, **dadurch gekennzeichnet dass,**
das Griffelement (24, 57) und das Verschlusselement derart entkoppelt sind, dass das Verschlusselement (26; 50) sowohl durch Bewegung des Griffelementes (24; 57) als auch unabhängig von einer Bewegung des Griffelementes (24; 57) in eine entsicherte Position bewegbar ist.

2. Medizinisches Instrument nach Anspruch 1, **dadurch gekennzeichnet, dass** die Rastverbindung zumindest ein bewegliches Rastelement (18; 44) aufweist und das Verschlusselement (26; 50) in einer gesicherten Position das Rastelement (18; 44) blockiert und in einer entsicherten Position das Rastelement (18; 44) freigibt.

3. Medizinisches Instrument nach Anspruch 2, **dadurch gekennzeichnet, dass** das Rastelement eine Rastkugel (44) ist.

4. Medizinisches Instrument nach Anspruch 2, **dadurch gekennzeichnet, dass** das Rastelement eine federnde Rastzunge (18) ist.

5. Medizinisches Instrument noch einem der Ansprüche 2 bis 4, **dadurch gekennzeichnet, dass** die Rastelemente (18; 44) in eine korrespondierende ringförmige Rastnut (22; 49)eingreifen.

6. Medizinisches Instrument nach einem der vorangehenden Ansprüche, **dadurch gekennzeichnet, dass** das Griffelement (24; 57) und das Verschfusselement (26; 50) in Richtung der Längsachse X des Instrumenteneinsatzes (4) bewegbar sind.

7. Medizinisches Instrument nach einem der vorangehenden Ansprüche, **dadurch gekennzeichnet, dass** das Verschlusselement (26; 50) durch Federkraft in der gesicherten Position gehalten wird.

8. Medizinisches Instrument nach einem der vorangehenden Ansprüche, **dadurch gekennzeichnet, dass** das Verschlusselement (26; 50) als Verschlusshülse und das Griffelement (24; 57) als Griffhülse ausgebildet sind, welche in Richtung der Längsachse X des Instrumenteneinsatzes (4) beweglich geführt sind, wobei die Verschlusshülse (26; 50) im Inneren der Griffhülse (24; 57) angeordnet ist.

9. Medizinisches Instrument nach Anspruch 8, **dadurch gekennzeichnet, dass** die Verschlusshülse (26; 50) zumindest eine Anlageschulter (34; 58) aufweist, welche von einer an der Griffhülse ausgebildeten Anlageschulter (36; 60) lediglich an einer in Bewegungsrichtung zu der entsicherten Position rückwärtigen Fläche hintergriffen wird.

10. Medizinisches Instrument nach einem der vorangehenden Ansprüche, **dadurch gekennzeichnet, dass** die Rastverbindung ein Rastelement (44) aufweist, welches zur Verbindung von Instrumenteneinsatz (4) und Instrumentenhandhabe (2) in eine korrespondierenden Rastnut (49) eingreift, welche axial einen gewellten oder gezackten Rand aufweist.

11. Medizinisches Instrument noch einem der vorangehenden Ansprüche, **dadurch gekennzeichnet, dass** das Griffelement (57) und das Verschlusselement (50) am proximalen Ende des Instrumenteneinsatzes (4) angeordnet sind.

12. Medizinisches Instrument nach einem der vorangehenden Ansprüche, **dadurch gekennzeichnet, dass** das Griffelement (57) drehfest mit dem Instrumenteneinsatz (4) verbunden ist.

13. Medizinisches Instrument nach einem der vorangehenden Ansprüche, **dadurch gekennzeichnet, dass** der Instrumenteneinsatz (4) ein Schaftrohr (6), eine im Inneren des Schaftrohres angeordnete Betätigungsstange (66) und zumindest ein am distalen Ende angeordnetes über die Betätigungsstange (66) bewegliches Element aufweist, wobei im Kraftfluss zur Bewegung des beweglichen Elementes zumindest ein Federelement (72) als Überlastsicherung angeordnet ist.

14. Medizinisches Instrument nach Anspruch 13, **dadurch gekennzeichnet, dass** das Schaftrohr (6) an seinem proximalen Ende in einem Verbindungselement (48) zur Verbindung mit einer Instrumentenhandhabe (2) gelagert ist und das Schaftrohr (6) sich in proximaler Richtung an dem Verbindungselement (48) über ein axial wirkendes Federelement (72) abstützt.

## Claims

1. A medical instrument with an instrument insert (4) and with an instrument handle (2) which are detachably connectable to one another via a locking connection, wherein a closure element (26; 50) for securing the locking connection as well as a grip element (24; 57) which must be moved for releasing the locking connection, are provided, **characterised in that** the grip element (24; 57) and the closure element are decoupled in a manner such that the closure element (26; 50) may be moved into an unsecured position by way of movement of the grip element (24; 57) as well as independently of a movement of the grip element (24; 57).

2. A medical instrument according to claim 1, **characterised in that** the locking connection comprises at least one movable locking element (18; 44), and the closure element (26; 50) in a secured position blocks the locking element (18; 44) and in an unsecured position releases the locking element (18; 44).

3. A medical instrument according to claim 2, **characterised in that** the locking element is a locking ball (44).

4. A medical instrument according to claim 2, **characterised in that** the locking element is a resilient locking tongue (18).

5. A medical instrument according to one of the claims 2 to 4, **characterised in that** the locking elements (18; 44) engage into a corresponding annular locking groove (22; 49).

6. A medical instrument according to one of the preceding claims, **characterised in that** the grip element (24; 57) and the closure element (26; 50) are movable in the direction of the longitudinal axis X of the instrument insert (4).

7. A medical instrument according to one of the preceding claims, **characterised in that** the closure element (26; 50) is held in the secured position by spring force.

8. A medical instrument according to one of the preceding claims, **characterised in that** the closure element (26; 50) is designed as a closure sleeve and the grip element (24; 57) as a grip sleeve, and these are movably guided in the direction of the longitudinal axis X of the instrument insert (4), wherein the closure sleeve (26; 50) is arranged in the inside of the grip sleeve (24; 57).

9. A medical instrument according to claim 8, **characterised in that** the closure sleeve (26; 50) comprises at least one contact shoulder (34; 58) behind which a contact shoulder (36; 60) formed on the grip sleeve engages only on a surface which is rearward in the movement direction to the unsecured position.

10. A medical instrument according to one of the preceding claims, **characterised in that** the locking connection comprises a locking element (44) which, for the connection of the instrument insert (4) and the instrument handle (2), engages into a corresponding locking groove (49) which axially comprises a waved or serrated edge.

11. A medical instrument according to one of the preceding claims, **characterised in that** the grip element (57) and the closure element (50) are arranged at the proximal end of the instrument insert (4).

12. A medical instrument according to one of the preceding claims, **characterised in that** the grip element (57) is connected to the instrument insert (4) in a rotationally fixed manner.

13. A medical instrument according to one of the preceding claims, **characterised in that** the instrument insert (4) comprises a shank tube (6), an actuation rod (66) arranged in the inside of the shank tube, and at least one element which is arranged at the distal end and is movable via the actuation rod (66), wherein at least one spring element (72), as an overload protection, is arranged in the transmission path for movement of the movable element.

14. A medical instrument according to claim 13, **characterised in that** the shank tube (6) at its proximal end is mounted in a connection element (48) for connection to an instrument handle (2), and the shank tube (6) in the proximal direction is supported on the connection element (48) via an axially acting spring element (72).

## Revendications

1. Dispositif médical comportant un insert d'instrument (4) et un élément de manipulation d'instrument (2), qui peuvent être reliés ensemble de façon amovible par un raccord à crans, dans lequel sont prévus un élément de blocage (26 ; 50) destiné à sécuriser le raccord à crans ainsi qu'un élément de poignée (24 ; 57) qui doit être déplacé pour supprimer le raccord à crans, **caractérisé en ce que** l'élément de poignée (24 ; 57) et l'élément de blocage sont découplés de façon telle que l'élément de blocage (26 ; 50) peut être déplacé dans une position déverrouillée tant par le déplacement de l'élément de poignée (24 ; 57) qu'indépendamment d'un déplacement de l'élément de poignée (24 ; 57).

2. Dispositif médical selon la revendication 1, **caractérisé en ce que** le raccord à crans présente au moins un élément d'arrêt mobile (18 ; 44) et **en ce que** l'élément de blocage (26 ; 50) dans une position sécurisée bloque l'élément d'arrêt (18 ; 44) et libère l'élément d'arrêt (18 ; 44) dans une position déverrouillée.

3. Dispositif médical selon la revendication 2, **caractérisé en ce que** l'élément d'arrêt est une bille d'arrêt (44).

4. Dispositif médical selon la revendication 2, **caractérisé en ce que** l'élément d'arrêt est une lame d'arrêt (18) résiliente.

5. Dispositif médical selon l'une des revendications 2 à 4, **caractérisé en ce que** les éléments d'arrêt (18 ; 44) s'engrènent dans une rainure d'arrêt annulaire (22 ; 49) correspondante.

6. Dispositif médical selon l'une des revendications précédentes, **caractérisé en ce que** l'élément de poignée (24 ; 57) et l'élément de blocage (26 ; 50) sont mobiles dans la direction de l'axe longitudinal X de l'insert d'instrument (4).

7. Dispositif médical selon l'une des revendications précédentes, **caractérisé en ce que** l'élément de blocage (26 ; 50) est maintenu en position sécurisée par effet de ressort.

8. Dispositif médical selon l'une des revendications précédentes, **caractérisé en ce que** l'élément de blocage (26 ; 50) est conçu sous la forme d'un manchon de blocage et l'élément de poignée (24 ; 57) sous la forme d'un manchon de préhension, qui sont guidés dans leur déplacement dans la direction de l'axe longitudinal X de l'insert d'instrument (4), le manchon de blocage (26 ; 50) étant disposé à l'intérieur du manchon de préhension (24 ; 57).

9. Dispositif médical selon la revendication 8, **caractérisé en ce que** le manchon de blocage (26 ; 50) présente au moins un épaulement de positionnement (34 ; 58) qui est pris à l'arrière par un épaulement de positionnement (36 ; 60) formé au niveau du manchon de préhension, uniquement au niveau d'une surface arrière par rapport à la position déverrouillée dans le sens du déplacement.

10. Dispositif médical selon l'une des revendications précédentes, **caractérisé en ce que** le raccord à crans présente un élément d'arrêt (44) qui s'engrène pour raccorder l'insert d'instrument (4) à l'élément de manipulation d'instrument (2) dans une rainure d'arrêt (49) correspondante qui présente au niveau axial un bord ondulé ou crénelé.

11. Dispositif médical selon l'une des revendications précédentes, **caractérisé en ce que** l'élément de poignée (57) et l'élément de blocage (50) sont disposés au niveau de l'extrémité proximale de l'insert d'instrument (4).

12. Dispositif médical selon l'une des revendications précédentes, **caractérisé en ce que** l'élément de poignée (57) est raccordé à l'insert d'instrument (4) sans pouvoir pivoter.

13. Dispositif médical selon l'une des revendications précédentes, **caractérisé en ce que** l'insert d'instrument (4) présente une tubulure (6), une tige d'actionnement (66) disposée à l'intérieur de la tubulure et au moins un élément disposé au niveau de l'extrémité distale et mobile via la tige d'actionnement (66), où dans le flux de force permettant le déplacement de l'élément mobile est disposé au moins un élément à ressort (72) en tant que limiteur de charge.

14. Dispositif médical selon la revendication 13, **caractérisé en ce que** la tubulure (6) est logée au niveau de son extrémité proximale dans un élément de raccordement (48) destiné au raccordement à un élément de manipulation d'instrument (2), et **en ce que** la tubulure (6) s'appuie dans la direction proximale contre l'élément de raccordement (48) via un élément à ressort (72) qui agit dans la direction axiale.
